# EUROPEAN PATENT APPLICATION

(11) **EP 4 834 480 A1**
(43) Date of publication of application: **04.11.2026**
(21) Application number: 25172880.4
(22) Date of filing: 28.04.2025
(51) Int. Cl.: B04B 1/08, B04B 11/02, B04B 11/06, C12M 1/26

(54) **CENTRIFUGAL SEPARATOR AND BIOPROCESSING SYSTEM AND METHOD**

(71) Applicant: Alfa Laval Corporate AB, 221 00 Lund (SE)
(72) Inventor: THORWID, Peter, 221 00 LUND (SE); EKSTRÖM, Anders, 221 00 LUND (SE); KÖNIGSSON, Staffan, 221 00 LUND (SE); LARSSON, Per-Gustaf, 221 00 LUND (SE)
(74) Representative: Alfa Laval Attorneys

(57) **Abstract**

The disclosure concerns a centrifugal separator, a bioprocessing system, and a method. The centrifugal separator comprises a stationary casing, a separator rotor (4) delimiting a separation space (8) and being arranged to rotate about a rotational axis (6) within the stationary casing, a separation aid (10) arranged in the separation space (8), an inlet (20) arranged in fluid communication with the separation space (8), a first outlet (22) arranged in fluid communication with a radially inner portion (26) of the separation space (8), and a second outlet (24) arranged in fluid communication with a radially outer portion (28) of the separation space (8). In a plane extending perpendicularly to the rotational axis (6), the separation aid (10) extends over a circular sector of < 360 degrees, such as < 180 degrees.

## Description

### TECHNICAL FIELD

The invention relates to a centrifugal separator, to a bioprocessing system, and to a method in a bioprocessing system.

### BACKGROUND

A centrifugal separator comprises a separator rotor arranged to rotate about a rotational axis. Inside the separator rotor there is a separation space wherein a separation aid, e.g. in the form of a stack of frustoconical separation discs is arranged. During operation of the centrifugal separator, a fluid feed mixture is fed into the separation space and the separation aid and is separated into at least a heavy phase and a light phase during rotation of the separator rotor. Heavy constituents of the fluid feed mixture travel under the influence of centrifugal force towards a periphery of the separation space, from where they are led out of the separation space forming at least part of the heavy phase. A light phase travels radially inwardly in the separation space, from where it is led out of the separation space. The light and heavy phases are continuously conducted from the separation space.

Centrifugal separators are highly efficient separation device due to the generated centrifugal force and the separation aid. Accordingly, large amounts of fluid feed mixture can be separated in an efficient manner in a centrifugal separator. Centrifugal separators are also compact in comparison with other separation devices such as e.g. filters and sedimentation tanks.

Recently it has been proposed to utilise centrifugal separators in bioprocessing systems.

For instance, WO 2022/128347 discloses a method for operating a bioprocessing system, which method includes: producing a cell culture mixture in a fermentor; conducting a flow of cell culture mixture from the fermentor into an interior of a centrifugal separator including a surface enlarging insert simultaneously with the step of producing the cell culture mixture; and returning continuously from the interior of the centrifugal separator a flow of liquid to the fermentor, simultaneously with both the steps of producing the cell culture mixture and conducting the flow of cell culture mixture.

The method discussed in WO 2022/128347 relates to so-called perfusion mode operation of a bioprocessing system. As opposed to batch operation, an operation method to which no fresh media is added and no used media and/or liquid is removed, and fed-batch operation wherein the process starts with a small media volume to which fresh medium is added and no liquid is removed until after termination of the process, perfusion mode operation relates to operation methods where the media is sequentially exchanged, fresh nutrients are sequentially added, and product i.e. used media is removed throughout the culture period.

Due to the use of the centrifugal separator in the bioprocessing system of WO 2022/128347, the system is compact and can be operated over long periods of time.

Traditionally, the separation efficiency of a centrifugal separator is changed e.g. by changing throughflow of process fluid through the centrifugal separator, wherein the term process fluid includes the fluid feed mixture, the separated light phase, and the separated heavy phase. Increasing the throughflow reduces separation efficiency but entails increasing the pressure of the process fluid. This may have negative consequences for certain kinds of process fluids, such as cell culture mixtures being processed in perfusion mode. Namely, the cells may be damaged by high pressure handling. A further option to change the separation efficiency of a centrifugal separator may be to reduce the rotational speed of the rotor of the centrifugal separator. This, however, still means that the separation space within the rotor is filled with process fluid and turnover of process fluid may be low, i.e. the time it takes for a quantity of process fluid to pass through the centrifugal separator is long. This, in turn, can be detrimental to some process fluids, such as cell culture mixtures being processed in perfusion mode. Selecting a smaller size centrifugal separator may be an option in some cases. However, centrifugal separators are offered in standard sizes for particular flow ranges of process fluid. For instance, for low flow rates, the number of available smaller sized centrifugal separators may be limited and commonly, a centrifugal separator is intended for a broad range of flow rates, which means that a particular centrifugal separator may not be suited for particular flow ranges in particular separation applications.

### SUMMARY

In some separation applications of fluid feed mixtures, small-sized particles are to be separated with the light phase and only particles above a certain limit size are to be separated with the heavy phase. In such separation applications, the use of centrifugal separators poses a problem since the small-sized particles risk being separated with the heavy phase, due to the high separation efficiency of the centrifugal separator.

It would be desirable to provide a specific separation efficiency in a centrifugal separator. To better address this, a centrifugal separator having the features defined in one of the independent claims is provided.

According to a first aspect, there is provided a centrifugal separator comprising a stationary casing, a separator rotor delimiting a separation space and being arranged to rotate about a rotational axis within the stationary casing, a separation aid arranged in the separation space, an inlet arranged in fluid communication with the separation space, a first outlet arranged in fluid communication with a radially inner portion of the separation space, and a second outlet arranged in fluid communication with a radially outer portion of the separation space. In a plane extending perpendicularly to the rotational axis, the separation aid extends over a circular sector of < 360 degrees, such as < 180 degrees.

Since the separation aid extends over a circular sector of < 360 degrees, such as < 180 degrees, the separation efficiency of the centrifugal separator is reduced compared to a centrifugal separator comprising a separation aid extending over full 360 degrees within the separation space.

More specifically, the separation efficiency of the centrifugal separator is determined by the size of the circular sector over which the separation aid extends. In the present centrifugal separator, a manufacturer can offer a specifically tailored separation aid and/or a specifically devised separator rotor with a specific separation aid in order to meet a particular separation efficiency, such as achieving a particular limit size for separated particles, the limit size being e.g. the smallest particle size that is separated with the heavy phase during use of the centrifugal separator, smaller particles being separated with the light phase.

Thus, the centrifugal separator can be utilised in certain processes where one or more of its other qualities are desired, such as e.g. its compact dimensions and/or its ability to be controlled for being used in different modes of a process.

Moreover, this separation aid extending over a circular sector of < 360 degrees enables a high turnover of process fluid within the centrifugal separator. Accordingly, the process fluid (feed mixture, light and heavy phases) enters and leaves the separator rotor within a shorter time frame than in a corresponding centrifugal separator comprising a full 360-degree separation aid. Namely, the < 360 degrees separation aid enables for the separation space to extends over a circular sector of < 360 degrees, which reduces its volume in comparison with a full 360-degree separation space.

According to a further aspect, there is provided an exchangeable separation insert for a centrifugal separator, the centrifugal separator comprising a base unit and the exchangeable separation insert mounted in the base unit, wherein at least part of the exchangeable separation insert forms part of a separator rotor of the centrifugal separator. The exchangeable separation insert comprises a rotor casing configured to rotate about a rotational axis and delimiting a separation space, a separation aid arranged in the separation space, an inlet arranged in fluid communication with the separation space, a first outlet arranged in fluid communication with a radially inner portion of the separation space, and a second outlet arranged in fluid communication with a radially outer portion of the separation space, wherein in a plane extending perpendicularly to the rotational axis, the separation aid extends over a circular sector of < 360 degrees, such as < 180 degrees.

Corresponding to the discussion above related to the centrifugal separator, since the separation aid extends over a circular sector of < 360 degrees, such as < 180 degrees, the separation efficiency of the exchangeable separation insert is reduced compared to an exchangeable separation insert comprising a separation aid extending over full 360 degrees within the separation space. Also, this separation aid extending over a circular sector of < 360 degrees enables a high turnover of process fluid within the exchangeable separation insert.

The exchangeable separation insert may be for a centrifugal separator according to the first aspect discussed above.

In embodiments of this aspect, a peripheral portion of the separation space is arranged radially outside the separation aid, and wherein the second outlet is arranged in fluid communication with the peripheral portion.

In embodiments of this aspect, the inlet comprises a supply conduit extending in a section of the separator rotor circumferentially outside the separation space.

In embodiments of this aspect, the second outlet comprises an outlet conduit extending in a section of the separator rotor circumferentially outside the separation space.

Furthermore, it would be desirable to provide a bioprocessing system enabling perfusion mode operation utilising a centrifugal separator. To better address this, one or more of a bioprocessing system, and a method in a bioprocessing system having the features defined in one or more of the independent claims is provided.

According to a further aspect, there is provided a bioprocessing system comprising a fermentor for producing therein a cell culture mixture, a centrifugal separator, a light phase receptacle, a mixture conduit system forming a connection between the fermentor and an inlet of the centrifugal separator, a heavy phase conduit system forming a connection between a heavy phase outlet of the centrifugal separator and the fermentor, a light phase conduit system connected to a light phase outlet of the centrifugal separator, to the light phase receptacle, and to the fermentor optionally, via the heavy phase conduit system, flow control equipment arranged in the mixture conduit system and the light phase conduit system, and a control arrangement configured for controlling the centrifugal separator and the flow control equipment. The control arrangement is configured to:
- conduct a cell culture mixture from the fermentor to the inlet of the centrifugal separator,
- in a first mode, separate the cell culture mixture into a light phase and a heavy phase in the centrifugal separator and conduct a flow of separated light phase from the light phase outlet of the centrifugal separator to the fermentor and a separated heavy phase from the heavy phase outlet to the fermentor, and
- in a second mode, separate the cell culture mixture into a light phase and a heavy phase in the centrifugal separator and conduct the flow of separated light phase from the light phase outlet to the light phase receptacle and the separated heavy phase from the heavy phase outlet to the fermentor.

Since the control arrangement is configured to operate in the first mode and the second mode as defined above, the bioprocessing system is configured for perfusion mode operation without having to stop flow of cell culture mixture via the centrifugal separator. Thus, during the first and second modes, stagnant conditions and thereto related deterioration of the cell culture mixture are avoided.

Namely, during first mode operation, the cell culture mixture is circulated through the centrifugal separator and is again returned to the fermentor in the form of the separated light and heavy phases, whereas during second mode operation, light phase is not returned to the fermentor and thus removed from the ongoing bioprocess.

According to a further aspect there is provided a method in a bioprocessing system, the bioprocessing system comprising a fermentor for producing a cell culture mixture therein, a centrifugal separator, and a light phase receptacle. The method comprises steps of:
- conducting a cell culture mixture from the fermentor to an inlet of the centrifugal separator, and

in a first mode:
   - separating the cell culture mixture into a light phase and a heavy phase in the centrifugal separator, and
   - conducting a flow of separated light phase from a light phase outlet of the centrifugal separator to the fermentor and a separated heavy phase from a heavy phase outlet of the centrifugal separator to the fermentor, and
in a second mode:
   - separating the cell culture mixture into a light phase and a heavy phase in the centrifugal separator, and
   - conducting the flow of separated light phase from the light phase outlet to the light phase receptacle and the separated heavy phase from the heavy phase outlet to the fermentor.

In a corresponding manner to the above discussion referring to the bioprocessing system, the method provides for perfusion mode operation of a bioprocessing system without having to stop flow of cell culture mixture via the centrifugal separator. Thus, during the first and second modes, stagnant conditions and thereto related deterioration of the cell culture mixture are avoided.

The centrifugal separator may be a high-speed centrifugal separator i.e., the separator rotor is rotatable at several thousand rotations per minute, such as at 3000 - 12000 rpm or higher, during use of the centrifugal separator.

However, the centrifugal separator may alternatively be operated at much lower rotational speed, less common for this type of centrifugal separator comprising a separator rotor with a separation aid arranged in the separation space. For instance, during separation operation of the centrifugal separator, the separator rotor may be rotated at 100 - 1500 rpm. At such low rotational speeds, imbalances of the separator rotor are less critical than in high-speed centrifugal separators and accordingly, may be acceptable to a larger extent.

The centrifugal separator comprises a drive arrangement for rotating the separator rotor about the rotational axis. The drive arrangement may comprise an electric motor and optionally, a transmission arranged between the electric motor and a rotor shaft, to which the separator rotor is connected.

The stationary casing may form a foundation that serves to support parts of the centrifugal separator, such as the separator rotor, a rotor shaft connected to the separator rotor, and the drive arrangement.

When the centrifugal separator is positioned for use thereof, the rotational axis may extend in a substantially vertical direction.

Herein, unless otherwise defined, the terms axial, radial, and circumferential relate to the rotational axis. An axial direction extends in parallel with the rotational axis of the centrifuge bowl, a radial direction extends perpendicularly to the rotational axis, and a circumferential direction extends at least partially around the rotational axis.

The separation aid forms a surface enlarging insert arranged in the separation space. The separation aid may comprise circular sector formed plates arranged one next to the other and at an angle < 90 degrees to the rotational axis, such as would be formed by a "slice" of a conventional stack of frustoconical separation discs, the "slice" being cut along two planes extending in parallel with, and including, the rotational axis. An alternative separation aid may comprise vanes extending along, and radially outwardly from, the rotational axis.

The separation space may extend 360 degrees around the rotational axis and accordingly, the separation aid may be provided in only a portion of the separation space.

Alternatively, in a plane extending perpendicularly to the rotational axis, the separation space may extend over a circular sector of < 360 degrees, such as < 180 degrees. The separation space may have an extension in a circumferential direction approximately the same as the separation aid, i.e. such that the separation aid fits into the separation space.

Thus, a smaller volume separation space may be provided in the centrifugal separator in comparison with a centrifugal separator comprising a separation space extending over 360 degrees around the rotational axis. This smaller volume enables high turnover of process fluid within the centrifugal separator, which may be beneficial e.g. in perfusion mode operation of bioprocessing systems for reducing the time each cell of the cell culture has to spend outside the fermentor.

The term process fluid includes the fluid feed mixture, the separated light phase, and the separated heavy phase.

The comparatively small volume of the separation space in the separator rotor of this embodiment entails that at any given time it will contain less process fluid than a full 360-degree separation space. This promotes high turnover of process fluid also at low flow rates.

During use of the centrifugal separator, separation of a fluid feed mixture is performed in the separation space of the centrifugal separator. The fluid feed mixture is led into the separation space via the inlet. A separated light phase is led out of the separation space via the first outlet, such as along the rotational axis or radially close to the rotational axis. A separated heavy phase is be led out of the separation space via the second outlet. The second outlet may extend from the radially outer portion of the separation space to the rotational axis or to a position close to the rotational axis.

The centrifugal separator may be configured for use in the field of pharmaceuticals, biopharmaceuticals, biotechnology, and thereto related fields. In the centrifugal separator, separation of substances from a fluid feed mixture, such as separation of cells from a cell culture mixture, may be performed in a sterile environment. For instance, the fluid feed mixture may be formed by a cell culture mixture such as a fermentation broth including a cell culture. The separated light phase may be formed by fermentation broth without the cells but with cell debris below a certain size, such as of a smaller size that the size of a representative cell size of the cell culture. During later stages of a culture period in a bioprocessing system, the cells of the cell culture mixture express an extracellular biomolecule such as an antibody or a protein, which then forms part of the light phase. The heavy phase may comprise the cells and cell debris suspended in fermentation broth.

The centrifugal separator may be configured for use in other technical fields, e.g. in fields where particles of the same chemical kind, structural kind, and/or density, but of different sizes, are to be separated with respective of the light and heavy phases. This may be referred to as classification. Thus, for instance, particles below a limit size may be separated with the light phase. Above the limit size, the particles may be separated with the heavy phase. Just to mention one example, such classification may be performed in a process step of kaolin/kaolinite production/extraction.

As discussed above, due to the reduced size of the present separation aid, in comparison with traditional separation aids extending 360 degrees around the rotational axis, a particular separation efficiency may be achieved during operation of the centrifugal separator.

The reduced size of the separation aid forms a further parameter affecting separation efficiency, e.g. the use of different rotational speeds for affecting separation efficiency is still available also in the present centrifugal separator with the reduced size separation aid.

The term separation efficiency relates to flow of fluid feed mixture that can be separated to a certain concentration of heavy phase and/or a certain clarity of light phase.

In the centrifugal separator, it may be possible to exchange at least part of the herein discussed separator rotor with a corresponding part for the separator rotor to comprise a 360-degree separation aid in an ordinary sized separation space and for higher efficiency separation.

Conversely, it may be possible to exchange at least part of a separator rotor comprising a 360 degree separation aid in an ordinary sized separation space with a corresponding part comprising the herein discussed separator rotor comprising a separation aid of < 360 degree extension, such as < 180 degree extension to reduce separation efficiency and separate comparatively larger particle sizes with the light phase.

The centrifugal separator may be a modular centrifugal separator, i.e. comprising a base unit and an exchangeable separation insert as discussed above. The exchangeable separation insert is an exchangeable preassembled separation insert.

The base unit may comprise the stationary casing and is configured for repeated use with different exchangeable separation inserts. That is, the base unit is reusable with new exchangeable parts such as the exchangeable separation insert after one process batch or after a limited number of process batches. The exchangeable separation insert may comprise the separation aid and at least a portion of the separator rotor, such as a radially inner portion of the separator rotor. The exchangeable separation insert may be configured for single use, i.e. for separation of one batch only or a limited number of batches of fluid feed mixture. The base unit may be configured for repeated use with different exchangeable separation inserts such as exchangeable separation inserts of the same kind or exchangeable separation inserts of different kinds, such as comprising differently sized separation aids.

In the bioprocessing system, a conduit system may be provided, the conduit system may comprise one or more of tubes, hoses, pipes, etc. configured for conducting liquid such as the cell culture mixture, separated light phase, and separated heavy phase within the bioprocessing system, and/or to and/or from external components connected to the bioprocessing system. Accordingly, the conduit system comprises the mixture conduit system, the light phase conduit system, and the heavy phase conduit system.

The flow control equipment may comprise one or more pumps and valves configured for pumping and directing liquid through the bioprocessing system, and/or to and/or from external components connected to the bioprocessing system.

The term control arrangement as used herein refers to a Programmable Logic Control (PLC), a Personal Computer (PC), an Embedded Computer (EC) or similar device integrating a Central-Processing-Unit (CPU) electronic device with calculating power. The control arrangement has various Input & Output (I/O) for the centrifugal separator and the flow control equipment. The control arrangement integrates software, algorithms, process recipe in build-in memory for controlling the centrifugal separator and the flow control equipment. Optionally, the control arrangement may also control aspects of the fermentor, e.g. for managing and/or analysis of the cultivation or fermentation process for ongoing process alignment according to the process recipe. The control arrangement may communicate with a variety of sensors and actuators in order to alter the process parameters.

As discussed above, the centrifugal separator may be utilised for efficient classification, or fractionation, of a cell culture mixture when separated in the centrifugal separator. That is, the largest particle size to be separated with a separated light phase by the centrifugal separator, the limit size, also referred to as critical particle, may be controlled by controlling the rotational speed of the separator rotor of the centrifugal separator and the flow of cell culture mixture into the centrifugal separator. Furthermore, a centrifugal separator comprising a separation aid as discussed herein enables further control over the limit size during a separation process.

Thus, for instance, the limit size may be set for undamaged whole cells to be include with the heavy phase and to exclude them from the light phase. Cell debris and other debris (which debris is smaller than whole cells) flows with both the light and heavy phases. Accordingly, in the second mode of operation, with separated light phase not being returned to the fermentor, also debris is removed from the fermentor, which removal is beneficial for the fermentation process in the fermentor.

The cell culture mixture is produced in the fermentor of the bioprocessing system. The term "fermentor" as used herein is intended to relate to any container wherein a cell culture mixture may be produced and may alternatively be referred to as a bioreactor or reactor. The fermentor may alternatively be referred to as fermenter. The fermentor may for instance be a so-called Stirred-Tank-Reactor" (STR) integrating an aeration device and agitation or mixing device(s) for forced exchange of nutrient and gas with the cells within the cell culture mixture.

Herein the term "bioprocess" is used for the process/es being performed in the bioprocessing system.

Fermentation and/or cultivation takes place in the fermentor during a step of producing the cell culture mixture therein. The terms "fermentation" and "cultivation" as used herein refers to hosting of micro-organisms or mammalian cells, such as living single-celled organisms, prokaryotes, eucaryotic cells, human cells, CHO, and bacteria for industrial purposes in a fermentor expressing an extracellular biomolecule. Fermentation / cultivation produces the cells of the cell culture mixture and the expressed biomolecule. The fermentation takes place over a culture period. The culture period may extend over several week during a large portion of which the product containing the extracellular biomolecule is produced, due to the perfusion mode operation of the bioprocessing system.

The fermentation may for example be for expression of an extracellular biomolecule, such as an antibody or a protein, from a mammalian cell culture mixture. Accordingly, the product contains the extracellular biomolecule. For instance, the cell culture mixture may comprise cells such as CHO (Chinese Hamster Ovary) cells and a growth medium composed to promote growth of the cells. CHO being a micro-organism and mammalian cell line very popular as expression platform of proteins for the pharma industry.

Further, in the fermentor, conditions for promoting growth of the cells are provided, e.g. by the addition of growth medium, oxygen, and maintenance of the cell culture mixture within a suitable temperature range.

The growth medium also referred to as culture medium is a liquid, the constituents of which promote growth of cells in the cell culture mixture and contains inter alia water, glucose, salts, and a source of amino acids. A gas such as clean air or oxygen is added to the growth medium e.g. in the fermentor.

The term "cell culture mixture" as used herein means the non-separated liquid content, the cultivation soup in a fermentor or bioreactor consisting of cells, debris, microorganisms, media with nutrients, waste, product, etc. During e.g. a first time period, the cell culture mixture may be separated into a light phase and a heavy phase in the centrifugal separator, whereby the cell density of the heavy phase increases over that of the entering cell culture mixture and as such is returned as to the fermentor and remixed with the cell culture mixture in the fermentor.

The term "product" is herein used for the media being expected to be produced or generated in the bioprocessing system. The light phase being separated in the centrifugal separator may form the product or may alternatively contain the product. The product may be subjected to downstream processing in order to extract therefrom a sought-after substance, such as an extracellular biomolecule, e.g. an antibody or a protein. That is, the product may be an intermediate result in a larger context of producing an end produce.

The term "cell/cells" as used herein is typically divided into: 1. living single-celled organisms, microbes such as; fungus, algae, moss, plankton, yeast, protozoa, eukaryotes, archaea, micro animals, extremophiles and plant cells or the like - 2. adherent or semi adherent or suspended living cells such as animal cells, insect cells, mammalian cells, human cells, stem cells - 3. prokaryotes and a variety of bacteria such as E.coli or the like. Most of the above may be genetically modified to provide a particular sought-after substance.

The term "perfusion" mode operation as used herein refers to the operation method or principle for a fermentor where the media is sequentially exchanged, fresh nutrients sequentially added, used media / product removed throughout a major portion of the culture period. The micro-organisms and mammalian cells retained in the perfusion mode operation may last typically 4 - 8 times longer than in batch operation and 2 - 4 times longer than in fed-batch operation. Mentioned purely as examples, perfusion mode operation may last a number of days, one or more weeks, or even one or more months.

Perfusion mode operation supports efficient facility utilisation by offering the possibility of using a smaller bioreactor /fermentor. Compared with batch or fed-batch processes, perfusion mode operation allows the cells of the cell culture mixture to stay in an exponential growth phase for an extended period of time and reach higher viable cell densities.

Moreover, the perfusion mode operation entails that the continuous return of the flow of liquid from the centrifugal separator to the fermentor and that the step of returning continuously from the interior of the centrifugal separator the flow of liquid to the fermentor, may take place over more than 90%, such as more than 95 %, of a duration of the entire culture period.

During early stages of a culture period in the bioprocess system, as an alternative to the first mode operation, for some duration, light phase may be disposed of, and the heavy phase is returned to the fermentor.

According to some embodiments of the centrifugal separator, a peripheral portion of the separation space may be arranged radially outside the separation aid. The second outlet may be arranged in fluid communication with the peripheral portion. In this manner, during operation of the centrifugal separator, the separated heavy phase, as separated into the peripheral portion may be continuously led out of the centrifugal separator, from the peripheral portion.

According to some embodiments, the inlet may comprise a supply conduit extending in a section of the separator rotor circumferentially outside the separation space. In this manner, a portion of the separator rotor may be utilised for conducting fluid feed mixture into the separation space. The herein discussed separation aid extending over a circular sector of < 360 degrees enables for the supply conduit to extend circumferentially outside the separation space.

In such embodiments, the separation space, in a plane extending perpendicularly to the rotational axis, may extend over a circular sector of < 360 degrees, such as < 180 degrees.

The term "circumferentially outside the separation space" refers to a position beside the separation space seen in a circumferential direction of the separator rotor.

According to some embodiments, the second outlet may comprise an outlet conduit extending in a section of the separator rotor circumferentially outside the separation space. In this manner, a portion of the separator rotor may be utilised for conducting separated heavy phase from the separation space. The herein discussed separation aid extending over a circular sector of < 360 degrees enables for the outlet conduit to extend circumferentially outside the separation space.

In such embodiments, the separation space, in a plane extending perpendicularly to the rotational axis, may extend over a circular sector of < 360 degrees, such as < 180 degrees.

According to some embodiments, the separation aid may comprise a first separation aid portion and a second separation aid portion. Seen in the plane extending perpendicularly to the rotational axis, each of the first and second separation aid portions may extend over a circular sector of < 180 degrees, such as < 90 degrees. In this manner, the separation aid may be provided in two portions of the separator rotor, such as in two diametrically opposite portions of the separator rotor. This may facilitate providing an evenly balanced separator rotor.

To/from, each of the first and second separation aid portions inlet conduits and outlet conduits may lead. For instance, the separation space may be divided into first and second separation space portions, each of which houses the respective first and second separation aid portions.

The inlet may be arranged in fluid communication with each of the first and second separation space portions, the first outlet may be arranged in fluid communication with a radially inner portion of each of the first and second separation space portions, and the second outlet may be arranged in fluid communication with a radially outer portion of each of the first and second separation space portions.

According to some embodiments of the bioprocessing system, the control arrangement may be configured to:
- in a third mode, conduct a flow of cell culture mixture from the light phase outlet to the fermentor and a flow of cell culture mixture from the heavy phase outlet to the fermentor. In this manner, unseparated cell culture mixture may pass through the centrifugal separator. Thus, stagnant conditions are avoided in the centrifugal separator, the mixture conduit system, the heavy phase conduit system, and at least part of the light phase conduit system during periods when no separation takes place in the centrifugal separator.

During perfusion mode operation of the bioprocessing system, the cell culture mixture may benefit from simply passing through the centrifugal separator, without being separated therein, and be returned to the fermentor. Thus, the cell culture mixture is not subjected to the forces associated with the act of separation in the centrifugal separator. Simultaneously, the risk of an amount of cell culture mixture deteriorating due to stagnant conditions in the centrifugal separator, in the mixture conduit system, in the heavy phase conduit system, and in the light phase conduit system, is avoided.

According to some embodiments, in the third mode, the centrifugal separator may be operated at a low rotational speed, such as below 50 rpm, and optionally a rotational direction of the rotor of the centrifugal separator may be altered at least once. In this manner, during third mode operation of the bioprocessing system, the passing of the cell culture mixture through the centrifugal separator may be facilitated and/or portions of the separation space where stagnant conditions may prevail may be avoided.

Further features of, and advantages with, the invention will become apparent when studying the appended claims and the following detailed description.

### BRIEF DESCRIPTION OF THE DRAWINGS

Various aspects and/or embodiments of the invention, including its particular features and advantages, will be readily understood from the examples discussed in the following detailed description and the accompanying drawings, in which:
Fig. 1 schematically illustrates an embodiment of a centrifugal separator,
Figs. 2a - 2c schematically illustrate views of a portion of a centrifugal separator,
Fig. 3 schematically illustrates a portion of a centrifugal separator according to an embodiment,
Fig. 4 schematically illustrates a centrifugal separator according to an embodiment,
Fig. 5 schematically illustrates a bioprocessing system according to an embodiment,
Fig. 6 illustrates a control arrangement,
Fig. 7 schematically illustrates embodiments of a method in a bioprocessing system, and
Fig. 8 illustrates embodiments of a computer-readable storage medium.

### DETAILED DESCRIPTION

Aspects and/or embodiments of the invention will now be described more fully. Like numbers refer to like elements throughout. Well-known functions or constructions will not necessarily be described in detail for brevity and/or clarity.

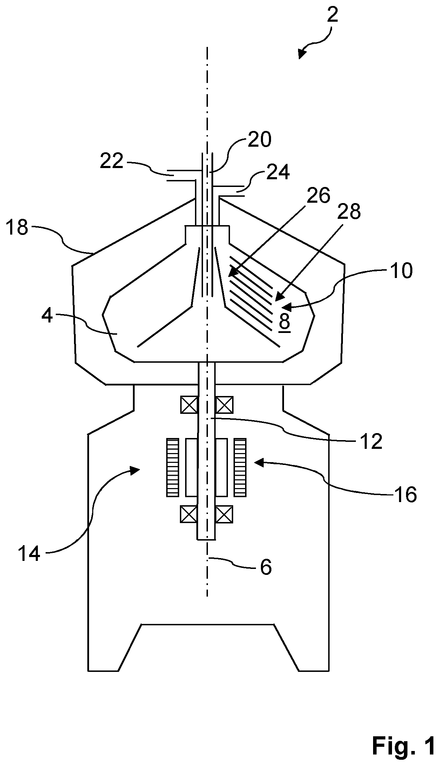

Fig. 1 schematically illustrates a section along a rotational axis 6 of an embodiment of a centrifugal separator 2. The centrifugal separator 2 is configured for separating a fluid feed mixture into a light phase and a heavy phase.

The centrifugal separator 2 comprises a separator rotor 4. The separator rotor 4 is arranged to rotate about the rotational axis 6. The separator rotor 4 delimits a separation space 8. A separation aid 10 is arranged in the separation space 8.

The centrifugal separator 2 further comprises a rotor shaft 12 connected to the separator rotor 4 and arranged to rotate with the separator rotor 4 about the rotational axis 6.

The rotor shaft 12 forms part of a drive arrangement 14 of the centrifugal separator 2, which drive arrangement 14 is arranged to rotate the separator rotor 4 about the rotational axis 6. In the illustrated embodiment, the drive arrangement 14 further comprises an electric motor 16. The rotor shaft 12 forms part of the electric motor 16, i.e. the separator rotor 4 is directly driven by the electric motor 16. Thus, the drive arrangement 14 may rotate the separator rotor 4 about the rotational axis 6.

Alternatively, the drive arrangement 14 may comprise a rotor shaft connected to the separator rotor 4, an electric motor, and a transmission arranged between the electric motor and the spindle.

The centrifugal separator 2 further comprises a stationary casing 18, which at least partially encloses the separator rotor 4.

The centrifugal separator 2 comprises an inlet 20 arranged in fluid communication with the separation space 8, a first outlet 22 arranged in fluid communication with a radially inner portion 26 of the separation space 8, and a second outlet 24 arranged in fluid communication with a radially outer portion 28 of the separation space 8.

In the illustrated embodiment, a portion of the inlet 20 extends concentrically with the rotational axis 6 into the separator rotor 4. Further, in the illustrated embodiment, a portion of the first outlet 22 extends radially outside the inlet 20 from the separator rotor 4, and a portion of the second outlet 24 extends radially outside the inlet 20 from the separator rotor 4. Alternative arrangements of the inlet 20, and the first and second outlets 22, 24 are foreseen, such as e.g. one or more of the inlet and the first and/or second outlets 22, 24 extending via a boring in the rotor shaft 12.

During separation use of the centrifugal separator 2, the separator rotor 4 is rotated by the drive arrangement 14 and a fluid feed mixture is conducted via the inlet 20 into the separation space 8. A separated light phase is conducted via the first outlet 22 from the separation space 8 and a separated heavy phase is conducted via the first outlet 22 from the separation space 8.

During separation of the fluid feed mixture in the centrifugal separator 2, the fluid feed mixture is led into the separation aid 10. The light phase being separated flows radially inwardly through the separation aid 10 towards the radially inner portion 26 of the separation space 8 and the rotational axis 6 and thereafter out of the separator rotor 4 via the first outlet 22. The separated heavy phase flows radially outwardly through the separation aid 10 towards the radially outer portion 28 of the separation space 8 and out of the separator rotor 4 via the second outlet 24.

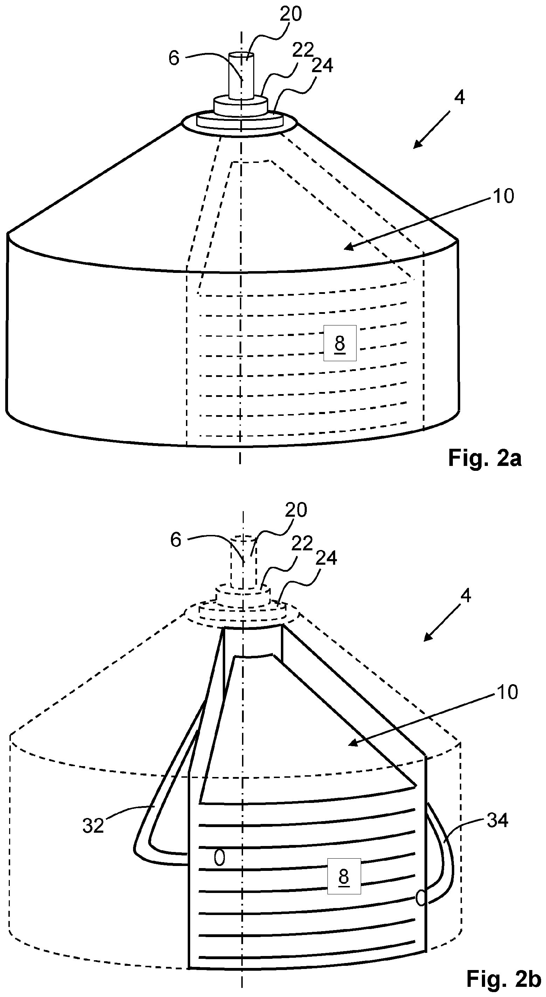

The herein discussed separation aid 10 has a specific characteristic in that it extends only partially around the rotational axis 6. Put differently, in a plane extending perpendicularly to the rotational axis 6, i.e. perpendicularly to the plane of **Fig. 1** and the therein indicated rotational axis 6, the separation aid 10 extends over a circular sector of < 360 degrees. For instance, the separation aid may extend over a circular sector of < 180 degrees. See further below with reference to **Figs. 2a** - **3****.**

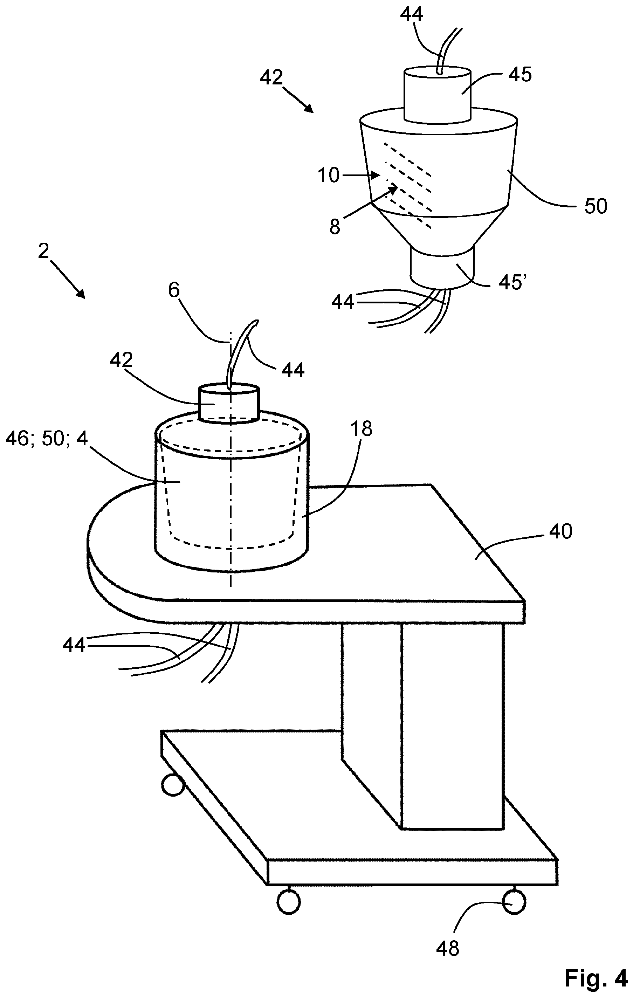

The centrifugal separator 2 including the separation aid 10 may be configured for long term use, such as in the centrifugal separator 2 shown in **Fig. 1****.** Alternatively, one or more portions of the centrifugal separator 2, such as a separation insert comprising the separation aid 10 may be configured to be exchangeable to enable single batch use, or limited number of batch use, (both herein also referred to as single use). See further with reference to the centrifugal separator 2 discussed with reference to **Fig. 4****.**

According to some embodiments, the centrifugal separator 2 may comprise a drive arrangement 14 configured to rotate the separator rotor 4 at a maximum speed of 1500 rpm. In this manner, certain separation operations may be performed while imbalance of the separator rotor 4 may be acceptable. This may be advantageous since the separation aid 10 arranged over < 360 degrees around the rotational axis 6 may be more difficult to balance well in comparison with a separation aid extending full 360 degrees around the rotational axis.

**Figs. 2a** - **2c** schematically illustrate views of a portion of a centrifugal separator. The centrifugal separator is a centrifugal separator 2 such as discussed with reference to **Figs. 1** **and** **4** comprising a separation aid 10 of the herein discussed kind. Accordingly, in the following reference is also made to the discussions of **Figs. 1** **and** **4****.**

Again, the centrifugal separator 2 comprises a separator rotor 4 delimiting a separation space 8 and being arranged to rotate about a rotational axis 6 within a stationary casing 18, and a separation aid 10 arranged in the separation space 8.

Again, an inlet 20 is arranged in fluid communication with the separation space 8, a first outlet 22 is arranged in fluid communication with a radially inner portion 26 of the separation space 8, and a second outlet 24 is arranged in fluid communication with a radially outer portion 28 of the separation space 8.

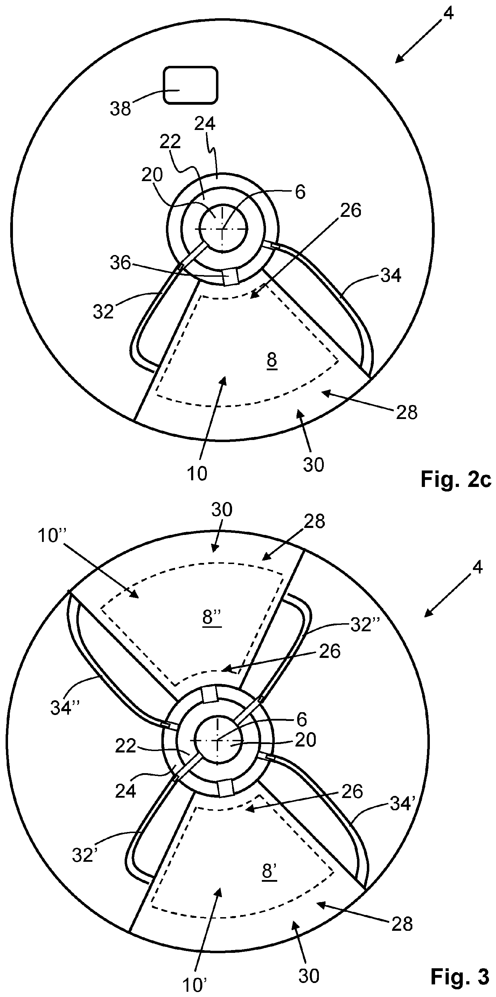

In **Fig. 2a****,** the separator rotor 4 is shown in a perspective view with the separation space 8 and the separation aid 10 arranged therein schematically indicated with broken lines. In **Fig. 2b****,** the separator rotor 4 is shown in a perspective view with an outer contour thereof indicated with broken lines and inter alia the separation space 8 and the separation aid 10 shown with solid lines. In **Fig. 2c****,** a top view of the separator rotor 4 is shown.

In a plane extending perpendicularly to the rotational axis 6, i.e. in the plane of **Fig. 2c****,** the separation aid 10 extends over a circular sector of < 360 degrees, such as < 180 degrees. The separation aid 10 may extend over a circular sector ≤ 120 degrees, such as over a circular sector ≤ 90 degrees.

In the embodiment of **Figs. 2a - 2c****,** the separation aid 10 extends over a circular sector of approximately 60 degrees.

In the embodiment of **Figs. 2a - 2c****,** the separation aid 10 comprises a stack of truncated circular sector shaped sheets, such as metal or plastic material sheets. The separation aid 10 is comparable to a radial "slice" of a commonly known stack of frustoconical separation discs.

Alternative embodiments of the separation aid 10 may comprise e.g. radial vanes or sheets extending in parallel with the rotational axis 6.

In the embodiments of **Figs. 2a - 2c****,** also the separation space 8 extends over a circular sector of < 360 degrees, such as < 180 degrees, in the plane extending perpendicularly to the rotational axis 6. The separation space 8 may extend over a circular sector ≤ 120 degrees, such as over a circular sector ≤ 90 degrees.

The separation space 8 may have an extension in a circumferential direction approximately the same as the separation aid 10, i.e. such that the separation aid 10 fits into the separation space 8. For instance, the separation space 8 may have an extension in a circumferential direction within a range of 1 - 30 % larger, such as 1 - 15% larger, than the extension of the separation aid 10 in the circumferential direction.

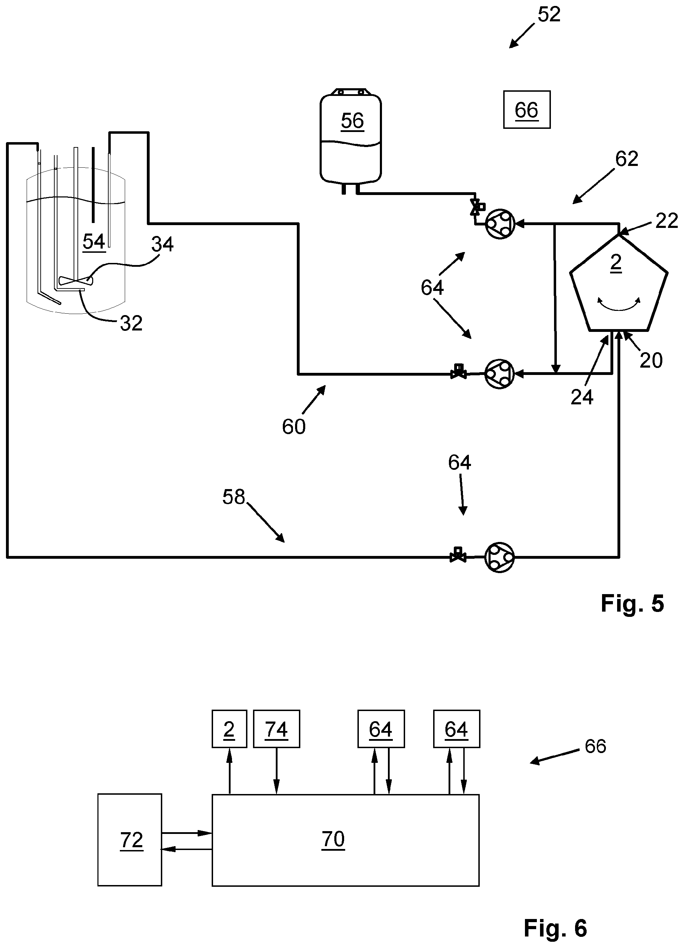

Accordingly, the separation space 8 may have a smaller volume than the separation space in a centrifugal separator of the same size but comprising a separation space extending 360 degrees around the rotational axis. The smaller volume enables high turnover of process fluid within the centrifugal separator 8, which may be beneficial e.g. in bioprocessing systems, such as the bio processing system 52 discussed below with reference to **Figs. 5 and 6****,** for reducing the time each cell of a cell culture mixture has to spend outside a fermentor of the system 52, in perfusion mode operation of the bioprocessing system 52.

The separator rotor 4 may comprise one or more solid body portions. Such solid body portions do not exhibit any spaces, such as the separation space 8 i.e., solid body portions are void of open volumes.

For instance, the separation space 8 may be formed in a solid body separator rotor 4. Accordingly, the separator rotor 4 will comprise at least one solid body portion with the separation space 8 delimited therefrom.

The inlet 20 comprise a supply conduit 32 extending in a section of the separator rotor 4 circumferentially outside the separation space 8. Thus, in a plane extending perpendicularly to the rotational axis 6, the supply conduit 32 is arranged in a circular sector of the separator rotor 4 adjacent to the separation space 8. During use of the centrifugal separator 2, a portion of the separator rotor 4 is utilised for the supply conduit 32 to conduct fluid feed mixture into the separation space 8.

For instance, the supply conduit 32 may be arranged in a circular sector within 45 degrees of the separation space 8 and/or the separation aid 10, in a plane extending perpendicularly to the rotational axis 6.

According to some embodiments, such as in the illustrated embodiment, the supply conduit 32 may extend in a direction having a radial component relative to the rotational axis 6. In this manner, the supply conduit 32 may extend from a radially inner portion of the separator rotor 4 to the separation space 8.

For instance, the supply conduit 32 may extend through a solid body portion of the separator rotor 4. That is, at least a portion of the supply conduit 32 may be provided as a bore or a cast channel through a solid body portion of the separator rotor 4.

In **Figs. 2b** **and** **2c****,** the supply conduit 32 is shown extending to a side portion of the separation space 8. There, the supply conduit 32 connects to the separation space 8 for establishing fluid communication between the inlet 20 and the separation space 8. The supply conduit 32 alternatively, may connect to any other suitable portion of the separation space 8, such as to an axial portion of the separation space 8.

A peripheral portion 30 of the separation space 8 is arranged radially outside the separation aid 10. The second outlet 24 is arranged in fluid communication with the peripheral portion 30 of the separation space 8. In this manner, during operation of the centrifugal separator 2, the separated heavy phase, as separated into the peripheral portion 30 may be continuously led out of the centrifugal separator, from the peripheral portion.

The second outlet 24 comprise an outlet conduit 34 extending in a section of the separator rotor 4 circumferentially outside the separation space 8. Thus, in a plane extending perpendicularly to the rotational axis 6, the outlet conduit 34 is arranged in a circular sector of the separator rotor 4 adjacent to the separation space 8. During use of the centrifugal separator 2, a portion of the separator rotor 4 is utilised for conducting separated heavy phase from the separation space 8.

For instance, the outlet conduit 34 may be arranged in a circular sector within 45 degrees of the separation space 8 and/or the separation aid 10, in a plane extending perpendicularly to the rotational axis 6.

According to some embodiments, such as in the illustrated embodiment, the outlet conduit 34 may extend in a direction having a radial component relative to the rotational axis 6. In this manner, the outlet conduit 34 may extend from the separation space 8, such as from its peripheral portion 30, to a radially inner portion of the separator rotor 4.

For instance, the outlet conduit 34 may extend through a solid body portion of the separator rotor 4. That is, at least a portion of the outlet conduit 34 may be provided as a bore or a cast channel through a solid body portion of the separator rotor 4.

In **Figs. 2b** **and** **2c****,** the outlet conduit 34 is shown extending from a side portion of the separation space 8. There, the outlet conduit 34 connects to the separation space 8 for establishing fluid communication between the peripheral portion 30 of the separation space 8 and the second outlet 24. Thus, during operation of the centrifugal separator 2, the separated heavy phase may be continuously led out of the separation space 8 from its peripheral portion 30.

The outlet conduit 34 alternatively, may connect to any other suitable portion of the separation space 8, such as to an axial portion of the separation space 8, as long as it enables fluid communication with the peripheral portion 30 of the separation space 8.

During operation of the centrifugal separator 2, the separated light phase is continuously led out of the separation space 8, from its radially inner portion 26 via the first outlet 22. One or more fluid passages 36 may be provided for this purpose between the radially inner portion 26 of the separation space 8 and the first outlet 22.

As indicated in **Fig. 2c****,** a balancing counterweight 38 may be provided in the separator rotor 4. Thus, imbalance of the separator rotor 4 may be at least reduced. The counterweight 38 may be established for the separation space 8 filled with process fluid.

**Fig. 3** schematically illustrates a portion of a centrifugal separator according to an embodiment. The centrifugal separator is a centrifugal separator 2 such as discussed with reference to **Figs. 1 - 2c****.** Accordingly, in the following reference is also made to the discussions of **Figs. 1 - 2c****.** The view of **Fig. 3** corresponds to that of **Fig. 2c****.** Mainly, the differentiating features of the **Fig. 3** embodiment are discussed in the following.

In the **Fig. 3** embodiment, the separation aid 10 comprise a first separation aid portion 10' and a second separation aid portion 10". Seen in a plane extending perpendicularly to the rotational axis 6, i.e. in the plane of **Fig. 3****,** each of the first and second separation aid portions 10', 10" extends over a circular sector of < 180 degrees, such as < 90 degrees.

In this manner, the separation aid 10', 10" is provided in two portions of the separator rotor 4, such as in two diametrically opposite portions of the separator rotor 4. This facilitates providing an evenly balanced separator rotor 4.

In the shown embodiment, the separation space 8 is divided into first and second separation space portions 8', 8". In each of the first and second separation space portions 8', 8" one of the first and second separation aid portions 10', 10" is arranged.

For the fluid feed mixture to enter the separation space portions 8', 8", the inlet 20 is arranged in fluid communication with the first and second separation space portions 8', 8", via first and second inlet conduits 32', 32" in a corresponding manner to that shown in the **Fig. 2c****.** For the light phase to leave the separation space portions 8', 8", the first outlet 22 is arranged in fluid communication with radially inner portions 26 of each of the first and second separation space portions 8', 8". For the heavy phase to leave the separation space portions 8', 8", the second outlet 24 is arranged in fluid communication with radially outer portions 28 of each of the first and second separation space portions 8', 8", via first and second outlet conduits 34', 34".

One or more interconnections between the first and second separation space portions 8', 8" may be provided. For instance, peripheral portions 30 of each of the first and second separation space portions 8', 8" may be arranged in fluid communication via one or more channels (not shown) extending at a periphery of the separator rotor 4.

**Fig. 4** schematically illustrates a centrifugal separator 2 according to an embodiment. The centrifugal separator 2 is a centrifugal separator 2 such as discussed with reference to **Figs.** 1 - 3. Accordingly, in the following reference is also made to the discussions of **Figs. 1 - 3****.** Mainly, the differentiating features of the **Fig. 4** embodiment are discussed in the following.

In the **Fig. 4** embodiment, the centrifugal separator 2 is a modular centrifugal separator 2. The modular centrifugal separator 2 comprises a base unit 40 and an exchangeable separation insert 42. In **Fig. 4** a first exchangeable separation insert 42 is mounted in the base unit 40 and a second exchangeable separation insert 42 is shown beside the centrifugal separator 2.

Thus, the centrifugal separator 2 is configured for single use. That is, after use, the first exchangeable separation insert 42 in the base unit 40 is exchanged with the second exchangeable separation insert 42. The first exchangeable separation insert 42 thereafter, may be disposed of. For instance, if the centrifugal separator 2 forms part of a bioprocessing system, e.g. as discussed below with reference to **Fig. 5****,** the first exchangeable separation insert 42 may be exchanged after one culture period.

During use of the modular centrifugal separator 2, the fluid feed mixture, the heavy phase, and the light phase only come into contact with the exchangeable separation insert 42. A conduit system configured for conducting the liquid feed mixture to the inlet 20 and separated phases from the first and second outlets 22, 24 also come into contact with the fluid feed mixture and the separated phases. The base unit 40 does not come into contact with the fluid feed mixture or any of the separated phases.

Tubes 44 connected to the exchangeable separation insert 42 may form part of the exchangeable separation insert 42. The tubes 44 form part of the above-mentioned conduit system 10 and lead to the inlet 20 and lead from respective outlets 22, 24 for separated light and heavy phases. Alternatively, one or more of the tubes 44 may form part of one or more exchangeable sets of tubes.

The base unit 40 comprises components for supporting and rotating at least part of the exchangeable separation insert 42. Thus, the base unit 40 comprises inter alia the stationary casing 18, a rotatable member 46, and the drive arrangement 14 (not shown) for rotating the rotatable member 46.

The stationary casing 18 is stationary during use of the modular centrifugal separator 2 and in relation to the base unit 40. However, the base unit 40 as such may be movable, e.g. in order to be positioned at different locations at a production facility of a user. For this purpose, the base unit 40 may be provided with wheels 48.

The exchangeable separation insert 42 is configured for part of it, such as a rotor casing 50 thereof, to be arranged inside an inner space of the rotatable member 46 of the base unit 40. Together, the rotor casing 50 of the exchangeable separation insert 42 and the rotatable member 46 of the base unit 40 form the separator rotor 4 of the centrifugal separator 2. The exchangeable separation insert 42 may also comprise one or more stationary portions 45, 45', such as portions where the tubes 44 connected to the exchangeable separation insert 42.

The tubes 44 may be arranged differently than in the illustrate embodiment. Any configuration of one, two, or three tubes 44 may be provided at a stationary part of the exchangeable separation insert 42. In the latter case, with three tubes at one stationary part, the exchangeable separation insert 42 may comprise only one stationary part, at its upper or lower end.

The separator rotor 4 of the centrifugal separator 2 is configured to rotate about the rotational axis 6. The rotor casing 50 of the exchangeable separation insert 42 delimits the separation space 8 therein. At least one of the first and second exchangeable separation inserts 42 comprises a reduced size separation aid 10 according to any one of aspects and/or embodiments discussed herein arranged in the separation space 8.

The base unit 40 may not be dedicated to use with only the herein described kind of exchangeable separation inserts 42. Also, exchangeable separation inserts of other kinds may be mounted in the base unit 40 to form a centrifugal separator, as long as outer dimensions of the relevant exchangeable separation insert fit in the base unit 40 and an interface between the exchangeable separation insert and the base unit 40 match.

The exchangeable separation insert 42 is an exchangeable preassembled separation insert 42, which accordingly is exchangeable as one component in the base unit 40. The exchangeable preassembled separation insert 42 comprises at least the rotor casing 50 and the separation aid 10 in the separation space 8.

**Fig. 4** shows embodiments of an exchangeable separation insert 42 for a centrifugal separator 2, the centrifugal separator 2 comprising a base unit 40 and the exchangeable separation insert 42 mounted in the base unit 40, wherein at least part of the exchangeable separation insert 42 forms part of a separator rotor 4 of the centrifugal separator 2.

The exchangeable separation insert 42 comprises a rotor casing 50 configured to rotate about a rotational axis 6 and delimiting a separation space 8, a separation aid 10 arranged in the separation space 8, an inlet 20 arranged in fluid communication with the separation space 8, a first outlet 22 arranged in fluid communication with a radially inner portion 26 of the separation space 8, and a second outlet 24 arranged in fluid communication with a radially outer portion 28 of the separation space 8. In a plane extending perpendicularly to the rotational axis 6, the separation aid 10 extends over a circular sector of < 360 degrees, such as < 180 degrees.

Similarly to the centrifugal separator 2 of **Figs. 1** - **3****,** which discussions again are referred to, the exchangeable separation insert 42 may be provided in one or more of the following embodiments:
According to some embodiments, a peripheral portion 30 of the separation space 8 is arranged radially outside the separation aid 10. The second outlet 24 is arranged in fluid communication with the peripheral portion 30.

According to some embodiments, the inlet 20 comprises a supply conduit 32 extending in a section of the separator rotor 4 circumferentially outside the separation space 8.

According to some embodiments, the supply conduit 32 extends in a direction having a radial component relative to the rotational axis 6.

According to some embodiments, the second outlet 24 comprises an outlet conduit 34 extending in a section of the separator rotor 4 circumferentially outside the separation space 8.

According to some embodiments, the outlet conduit 34 extends in a direction having a radial component relative to the rotational axis 6.

According to some embodiments, the separation aid 10 comprises a first separation aid portion 10' and a second separation aid portion 10". Seen in the plane extending perpendicularly to the rotational axis 6, each of the first and second separation aid portions 10', 10" extends over a circular sector of < 180 degrees, such as < 90 degrees.

Fig. 5 schematically illustrates a bioprocessing system 52 according to an embodiment.

The bioprocessing system 52 comprises a fermentor 54 for producing therein a cell culture mixture, a centrifugal separator 2, a light phase receptacle 56, a mixture conduit system 58, a heavy phase conduit system 60, a light phase conduit system 62, flow control equipment 64, and a control arrangement 66 configured for controlling the centrifugal separator 2 and the flow control equipment 64.

The light phase receptacle 56 is configured for receiving a light phase separated in the centrifugal separator 2. The light phase receptacle 56 may be configured for intermediate storage of the separated light phase.

The mixture conduit system 58 forms a connection between the fermentor 54 and an inlet 20 of the centrifugal separator 2. The heavy phase conduit system 60 forms a connection between a heavy phase outlet 24 of the centrifugal separator 2 and the fermentor 54. The light phase conduit system 62 is connected to a light phase outlet 22 of the centrifugal separator 2, to the light phase receptacle 56, and to the fermentor 54. Optionally, the connection of the light phase conduit system 62 may be connected to the fermentor 54 via the heavy phase conduit system 60.

The flow control equipment 64 is arranged at least in the mixture conduit system 58 and the light phase conduit system 62.

The centrifugal separator 2 may be a centrifugal separator 2 as discussed herein e.g. with reference to **Figs. 1** - **4****.** Accordingly, in the following reference is also made to the discussion of **Figs. 1 - 4****.**

According to some embodiments the centrifugal separator 2 may be configured for single use operation and may comprise an exchangeable separation insert. According to alternative embodiments, the centrifugal separator 2 may be configured for permanent and/or long-term use. In such embodiments, the centrifugal separator 2 may be cleaned in between culture periods.

The conduit systems 58, 60, 62 are arranged to interconnect the fermentor 54, the centrifugal separator 2, and the light phase receptacle 56. The flow control equipment 64 is arranged in the at least two of the conduit systems 58, 60, 62. The flow control equipment 64 may comprise one or more pumps and/or one or more valves. The flow control equipment 64 is arranged to control flow of one or more of the cell culture mixture, the heavy phase separated in the centrifugal separator 2, and the light phase separated in the centrifugal separator 2. Also, the centrifugal separator 2, may have a flow controlling function in the conduit system 10, e.g. when the separator rotor 4 thereof is rotating.

Further conduits and/or conduit systems may be provided in the bioprocess system 52, e.g. for disposing of excess fluid during one or more stages of the culture period.

The flow control equipment 64 may comprise one or more of a feed pump arranged in the mixture conduit system 58, a heavy phase pump arranged in the heavy phase conduit system 60, a light phase pump arranged in the light phase conduit system 62. The feed pump may be utilised for transporting cell culture mixture from the fermentor 54 to the centrifugal separator 2. The heavy phase pump may be utilised for transporting separated heavy phase back to the fermentor 54. According to some embodiments, the heavy phase pump may be utilised for transporting the cell culture mixture from the centrifugal separator 2 back to the fermentor 54. According to some embodiments, the heavy phase pump may be utilised for transporting separated light phase from the centrifugal separator 2 back to the fermentor 54. The light phase pump may be utilised for transporting separated light phase to the light phase receptacle 56. Also, the centrifugal separator 2 as such may have a pumping effect on one or more of the cell culture mixture and the separated light phase.

The flow control equipment 64 may comprise one or more of a first valve arrangement arranged in the mixture conduit system 58, a second valve arrangement arranged in the heavy phase conduit system 60, and a third valve arrangement arranged in the light phase conduit system 62. In this manner, further control over the liquids flowing in the bioprocessing system 52 may be provided. For instance, the first valve arrangement may control the flow of cell culture mixture from the fermentor 54 to the centrifugal separator 2. The second valve arrangement may control the flow of heavy phase and/or the cell culture mixture and/or the light phase from the centrifugal separator 2 back to the fermentor 54. The third valve arrangement may control the flow of light phase from the centrifugal separator 2 to the receiving container 8 and/or to the heavy phase conduit system 60.

The feed pump may be a centrifugal pump or a peristaltic pump, and each of the heavy phase pump and the light phase pump may be a peristaltic pump. In this manner, gentle handling of the cells in the cell culture mixture and the heavy phase may be provided by the pumps, in particular when operated at low rotational speeds. Thus, the cell culture mixture and the heavy phase may be subjected to low shear forces to not damaging cells of the cell culture mixture. The peristaltic pumps may function as precise flow control devices. The peristaltic pumps may also function as flowmeters and thus, provide flow data for e.g. one or more of the separated light and heavy phases.

The control arrangement 66 is configured for controlling the centrifugal separator 2 and at least part of the flow control equipment 64.

The control arrangement 66 may be configured to control the fermentor 54, such as a pump (not shown) of the fermentor 54, a valve (not shown) of the fermentor 54, an air inlet 32 to the fermentor 54, and/or a stirring member 34 arranged in the fermentor 54. In this manner, the control arrangement 66 may also control at least some aspects of the cell culture production in the bioprocessing system 52.

Alternatively, the control arrangement 66 may be configured for controlling the centrifugal separator 2 and the flow of liquids to and from the centrifugal separator 2. The fermentor 54 and the culture process therein may be controlled by a separate control arrangement. In such embodiments, the control arrangement 66 of the centrifugal separator 2 and the flow control equipment 64 may communicate with the separate control arrangement of the fermentor 54.

The bioprocessing system 52 is configured for perfusion mode operation utilising the centrifugal separator 2.

**Fig. 6** illustrates a control arrangement 66 according to embodiments to be utilised in connection with the different aspects and/or embodiments of the herein discussed bioprocessing system 52, e.g. with reference to **Fig. 5****.** In following reference is also made to the discussions of **Figs. 1 - 5****.**

The control arrangement 66 comprises at least one calculation unit 70, which may take the form of substantially any suitable type of processor circuit or microcomputer, e.g. a circuit for digital signal processing (digital signal processor, DSP), a Central Processing Unit (CPU), a processing unit, a processing circuit, a processor, an Application Specific Integrated Circuit (ASIC), a microprocessor, or other processing logic that may interpret and execute instructions.

The control arrangement 66 comprises a memory unit 72. The calculation unit 70 is connected to the memory unit 72, which provides the calculation unit 70 with, e.g. stored programme code, data tables, and/or other stored data which the calculation unit 70 requires to enable it to do calculations and to control the centrifugal separator 2 and the control equipment 64. The calculation unit 70 is also adapted to store partial or final results of calculations in the memory unit 72. The memory unit 72 may comprise a physical device utilised to store data or programs, i.e. sequences of instructions on a temporary or permanent basis. According to some embodiments, the memory unit 72 may comprise integrated circuits comprising silicon-based transistors. The memory unit 72 may comprise e.g. a memory card, a flash memory, a USB memory, a hard disc, or another similar volatile or non-volatile storage unit for storing data such as e.g. ROM (Read-Only Memory), PROM (Programmable Read-Only Memory), EPROM (Erasable PROM), EEPROM (Electrically Erasable PROM), etc. in different embodiments.

The control arrangement 66 may further comprise one or more sensors 74. As mentioned above, also peristaltic pumps of the control equipment 64 may provide data, such as flow data. The calculation unit 70 receives measured data, such as e.g. centrifugal separator rotational speed from the one or more sensors 74 and/or flow data from peristaltic pumps. The control arrangement 66 sends control data to the centrifugal separator 2 and the control equipment 64 for controlling e.g. one or more of centrifugal separator rotational speed, pump speeds, and valve arrangement settings.

Thus, the calculation unit 70 is configured to receiving input signals from e.g. the sensor/s 74 and to send output signals to the centrifugal separator 2 and the control equipment 64. These signals may comprise waveforms, pulses or other attributes, which can be detect and sent as information by the calculation unit 70, and which can be directly or indirectly converted to signals processable by the calculation unit 70. Each of connections to the sensor/s, the centrifugal separator 2, and the control equipment 64 may take the form of one or more from among a cable, a data bus, e.g. a CAN (controller area network) bus, a MOST (media orientated systems transport) bus or some other bus configuration, or a wireless connection. In the embodiment depicted, only one calculation unit 70 and memory 72 are shown, but the control arrangement 66 may alternatively comprise more than one calculation unit and/or memory.

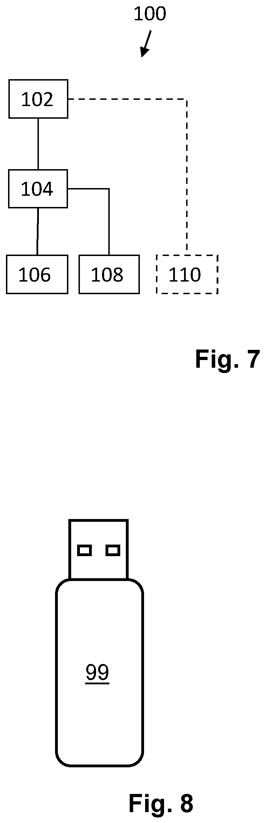

In the following, the control arrangement 66 and its operation is discussed, inter alia with reference to Figs. 1 - 6. Moreover, the control arrangement 66 may be configured to perform embodiments of a method 100 as discussed herein, inter alia with reference to Fig. 7. Hence, advantages discussed in connection with the method 100 may be applicable in the bioprocessing system 52 and vice versa.

The control arrangement 66 is configured to:
- Conduct a cell culture mixture from the fermentor 54 to the inlet 20 of the centrifugal separator 2.
- In a first mode, separate the cell culture mixture into a light phase and a heavy phase in the centrifugal separator 2 and conduct a flow of separated light phase from the light phase outlet 22 of the centrifugal separator 2 to the fermentor 54 and a separated heavy phase from the heavy phase outlet 24 to the fermentor 54.
- In a second mode, separate the cell culture mixture into a light phase and a heavy phase in the centrifugal separator 2 and conduct the flow of separated light phase from the light phase outlet to the light phase receptacle 56 and the separated heavy phase from the heavy phase outlet to the fermentor 54.

In this manner, the bioprocessing system 52 is configured for perfusion mode operation without having to stop flow of cell culture mixture via the centrifugal separator 2 and stagnant conditions are avoided during the first and second modes. Specifically, stagnant conditions are avoided in the mixture conduit system 58 and the heavy phase conduit system 60, where living cells of the cell culture are transported from, and to, the fermentor 54. Stagnant conditions in the light phase conduit system 62, wherein light phase without living cells is transported therein, may be accepted.

According to some embodiments of the bioprocessing system 52, the control arrangement 66 may be configured to:
- in a third mode, conduct a flow of cell culture mixture from the light phase outlet 22 to the fermentor 54 and a flow of cell culture mixture from the heavy phase outlet 24 to the fermentor 54.

Thus, unseparated cell culture mixture may pass through the centrifugal separator 2. Thus, stagnant conditions are avoided in the centrifugal separator 2, the mixture conduit system 58, the heavy phase conduit system 60, and at least part of the light phase conduit system 62 during periods when no separation takes place in the centrifugal separator 2.

By simply passing through the centrifugal separator 2, without being separated therein, and being returned to the fermentor 54, the cell culture mixture is not subjected to separation forces in the centrifugal separator 2 while stagnant conditions in the centrifugal separator 2, in the mixture conduit system 58, in the heavy phase conduit system 60, and in at least part of the light phase conduit system 62, is avoided.

Concerning the light phase conduit system 62, as indicated in **Fig. 5****,** only part of it may be flowed through by cell culture mixture during third mode operation namely, through that part of the light phase conduit system 62 connecting the light phase outlet 22 with the heavy phase conduit system 60. Other parts of the light phase conduit system 62, leading to the light phase receptacle 56, are only flowed through by separated light phase and thus, stagnant conditions in such parts are not detrimental to the cells of the cell culture mixture.

According to some embodiments, in the third mode, the centrifugal separator 2 may be operated at a low rotational speed, such as below 50 rpm, and optionally a rotational direction of the separator rotor 4 of the centrifugal separator 2 may be altered at least once.

Thus, during third mode operation of the bioprocessing system 52, the passing of the cell culture mixture through the centrifugal separator 2 may be facilitated and/or portions of the separation space 8 where stagnant conditions may prevail may be avoided.

According to some embodiments, the centrifugal separator 2 may be a centrifugal separator 2 according to any one of the aspects and or embodiments discussed herein, or the centrifugal separator 2 may comprise an exchangeable separation insert 42 according to any one of aspects and/or embodiments discussed herein. In this manner, the bioprocessing system 52, may be operated with a centrifugal separator 2 providing a separation efficiency suitable for the separation of cells from a cell culture mixture.

**Fig. 7** schematically illustrates embodiments of a method 100 in a bioprocessing system. The bioprocessing system may be a bio processing system 52 as discussed above with reference to **Figs. 5 and 6****.** In the following reference is also made to the discussion of **Figs. 1 - 6****.**

Again, the bioprocessing system 52 comprises a fermentor 54 for producing a cell culture mixture therein, a centrifugal separator 2, and a light phase receptacle 56.

The method 100 comprises steps of:
- Conducting 102 a cell culture mixture from the fermentor 54 to an inlet 20 of the centrifugal separator 2, and

in a first mode:
   - Separating 104 the cell culture mixture into a light phase and a heavy phase in the centrifugal separator 2, and
   - conducting 106 a flow of separated light phase from a light phase outlet 22 of the centrifugal separator 2 to the fermentor 54 and a separated heavy phase from a heavy phase outlet 24 of the centrifugal separator 2 to the fermentor 54, and
in a second mode:
   - Separating 104 the cell culture mixture into a light phase and a heavy phase in the centrifugal separator 2, and
   - conducting 108 the flow of separated light phase from the light phase outlet 22 to the light phase receptacle 56 and the separated heavy phase from the heavy phase outlet 24 to the fermentor 54.

According to some embodiments, the method 100 may comprise a step of:
- conducting 110, in a third mode, a flow of cell culture mixture from the light phase outlet 22 to the fermentor 54 and a flow of cell culture mixture from the heavy phase outlet 24 to the fermentor 54. In this manner, as an alternative to the first and second modes, the method 100 in the third mode, may enable unseparated cell culture mixture may pass through the centrifugal separator 2. Thus, stagnant conditions are avoided in the centrifugal separator 2, the mixture conduit system 58, the heavy phase conduit system 60, and at least part of the light phase conduit system 62 during periods when no separation takes place in the centrifugal separator 2.

According to some embodiments of the method 100, in the third mode, the centrifugal separator 2 may be operated at a low rotational speed, such as below 50 rpm, and optionally a rotational direction of the rotor 4 of the centrifugal separator 2 may be altered at least once. In this manner, during third mode operation of the bioprocessing system 52, the passing of the cell culture mixture through the centrifugal separator 2 may be facilitated and/or portions of the separation space 8 where stagnant conditions may prevail may be avoided.

According to some embodiments of the method 100, the centrifugal separator 2 may a centrifugal separator 2 according to any one of aspects and/or embodiments discussed herein, or the centrifugal separator 2 may comprise an exchangeable separation insert 42 according to any one of aspects and/or embodiments discussed herein. In this manner, the bioprocessing system 52, may be operated with a centrifugal separator 2 providing a separation efficiency suitable for the separation of cells from a cell culture mixture.

According to a further aspect, there is provided a computer program comprising instructions which, when the program is executed by a computer or a calculation unit 70, causes the computer or the calculation unit 70 to carry out a method 100 according to any one of aspects and/or embodiments discussed herein.

One skilled in the art will appreciate that the method 100 in the bioprocessing system 52 may be implemented by programmed instructions. These programmed instructions are typically constituted by a computer program, which, when it is executed in a computer or calculation unit 70, ensures that the computer or calculation unit 70 carries out the desired control, such as the above discussed method steps 102 - 110. The computer program is usually part of a computer-readable storage medium which comprises a suitable digital storage medium on which the computer program is stored.

Fig. 8 illustrates embodiments of a computer-readable storage medium 99 comprising instructions which, when executed by a computer or calculation unit 70, cause the computer or calculation unit 70 to carry out the steps of the method 100 according to any one of aspects and/or embodiments discussed herein.

The computer-readable storage medium 99 may be provided for instance in the form of a data carrier carrying computer program code for performing at least some of the steps 102 - 110 according to some embodiments when being loaded into the one or more calculation units 70. The data carrier may be, e.g. a ROM (read-only memory), a PROM (programable read-only memory), an EPROM (erasable PROM), a flash memory, an EEPROM (electrically erasable PROM), a hard disc, a CD ROM disc, a memory stick, an optical storage device, a magnetic storage device or any other appropriate medium such as a disk or tape that may hold machine readable data in a non-transitory manner. The computer-readable storage medium may furthermore be provided as computer program code on a server and may be downloaded to the calculation unit 70 remotely, e.g., over an Internet or an intranet connection, or via other wired or wireless communication systems.

The computer-readable storage medium 99 shown in **Fig. 8** is a nonlimiting example in the form of a USB memory stick.

The terminology used herein is for the purpose of describing particular aspects only and is not intended to be limiting of the disclosure. As used herein, the singular forms "a," "an," and "the" are intended to include the plural forms as well, unless the context clearly indicates otherwise. As used herein, the term "and/or" includes any and all combinations of one or more of the associated listed items. It will be further understood that the terms "comprises," "comprising," "includes," and/or "including" when used herein specify the presence of stated features, integers, actions, steps, operations, elements, and/or components, but do not preclude the presence or addition of one or more other features, integers, actions, steps, operations, elements, components, and/or groups thereof.

It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure.

Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein.

It is to be understood that the foregoing is illustrative of various examples and that the invention is defined only by the appended claims. A person skilled in the art will realize that the examples may be modified, and that different features of the examples may be combined to create examples other than those described herein, without departing from the scope of the invention, as defined by the appended claims.

## Claims

1. A centrifugal separator (2) comprising a stationary casing (18), a separator rotor (4) delimiting a separation space (8) and being arranged to rotate about a rotational axis (6) within the stationary casing (18), a separation aid (10) arranged in the separation space (8), an inlet (20) arranged in fluid communication with the separation space (8), a first outlet (22) arranged in fluid communication with a radially inner portion (26) of the separation space (8), and a second outlet (24) arranged in fluid communication with a radially outer portion (28) of the separation space (8), wherein
in a plane extending perpendicularly to the rotational axis (6), the separation aid (10) extends over a circular sector of < 360 degrees, such as < 180 degrees.

2. The centrifugal separator (2) according to claim 1, wherein a peripheral portion (30) of the separation space (8) is arranged radially outside the separation aid (10), and wherein the second outlet (24) is arranged in fluid communication with the peripheral portion (30).

3. The centrifugal separator (2) according to claim 1 or 2, wherein the inlet (20) comprises a supply conduit (32) extending in a section of the separator rotor (4) circumferentially outside the separation space (8).

4. The centrifugal separator (2) according to claim 3, wherein the supply conduit (32) extends in a direction having a radial component relative to the rotational axis (6).

5. The centrifugal separator (2) according to any one of the preceding claims, wherein the second outlet (24) comprises an outlet conduit (34) extending in a section of the separator rotor (4) circumferentially outside the separation space (8).

6. The centrifugal separator (2) according to claim 5, wherein the outlet conduit (34) extends in a direction having a radial component relative to the rotational axis (6).

7. The centrifugal separator (2) according to any one of the preceding claims, wherein the separation aid (10) comprises a first separation aid portion (10') and a second separation aid portion (10"), and wherein seen in the plane extending perpendicularly to the rotational axis (6), each of the first and second separation aid portions (10', 10") extends over a circular sector of < 180 degrees, such as < 90 degrees.

8. The centrifugal separator (2) according to any one of the preceding claims, comprising a drive arrangement (14), wherein the drive arrangement (14) is configured to rotate the separator rotor (4) at a maximum speed of 1500 rpm.

9. An exchangeable separation insert (42) for a centrifugal separator (2), the centrifugal separator (2) comprising a base unit (40) and the exchangeable separation insert (42) mounted in the base unit (40), wherein at least part of the exchangeable separation insert (42) forms part of a separator rotor (4) of the centrifugal separator (2), wherein
the exchangeable separation insert (42) comprises a rotor casing (50) configured to rotate about a rotational axis (6) and delimiting a separation space (8), a separation aid (10) arranged in the separation space (8), an inlet (20) arranged in fluid communication with the separation space (8), a first outlet (22) arranged in fluid communication with a radially inner portion (26) of the separation space (8), and a second outlet (24) arranged in fluid communication with a radially outer portion (28) of the separation space (8), wherein
in a plane extending perpendicularly to the rotational axis (6), the separation aid (10) extends over a circular sector of < 360 degrees, such as < 180 degrees.

10. A bioprocessing system (52) comprising
a fermentor (54) for producing therein a cell culture mixture,
a centrifugal separator (2),
a light phase receptacle (56),
a mixture conduit system (58) forming a connection between the fermentor (54) and an inlet (20) of the centrifugal separator (2),
a heavy phase conduit system (60) forming a connection between a heavy phase outlet (24) of the centrifugal separator (2) and the fermentor (54),
a light phase conduit system (62) connected to a light phase outlet (62) of the centrifugal separator (2), to the light phase receptacle (56), and to the fermentor (54) optionally, via the heavy phase conduit system (60),
flow control equipment (64) arranged in the mixture conduit system (58) and the light phase conduit system (62), and
a control arrangement (66) configured for controlling the centrifugal separator (2) and the flow control equipment (64), wherein
the control arrangement (66) is configured to:
- conduct a cell culture mixture from the fermentor (54) to the inlet (20) of the centrifugal separator (2),
- in a first mode, separate the cell culture mixture into a light phase and a heavy phase in the centrifugal separator (2) and conduct a flow of separated light phase from the light phase outlet (22) of the centrifugal separator (2) to the fermentor (54) and a separated heavy phase from the heavy phase outlet (24) to the fermentor (54), and
- in a second mode, separate the cell culture mixture into a light phase and a heavy phase in the centrifugal separator (2) and conduct the flow of separated light phase from the light phase outlet (22) to the light phase receptacle (56) and the separated heavy phase from the heavy phase outlet (24) to the fermentor (54).

11. The bioprocessing system (52) according to claim 10, wherein the control arrangement (66) is configured to:
- in a third mode, conduct a flow of cell culture mixture from the light phase outlet (22) to the fermentor (54) and a flow of cell culture mixture from the heavy phase outlet (24) to the fermentor (54).

12. The bioprocessing system (52) according to claim 11, wherein in the third mode, the centrifugal separator (2) is operated at a low rotational speed, such as below 50 rpm, and optionally a rotational direction of the rotor (4) of the centrifugal separator (2) is altered at least once.

13. The bioprocessing system (52) according to any one of claims 10 - 12, wherein the centrifugal separator (2) is a centrifugal separator (2) according to any one of claims 1 - 8 or a centrifugal separator (2) comprising an exchangeable separation insert (42) according to claim 9.

14. A method (100) in a bioprocessing system (52), the bioprocessing system (52) comprising a fermentor (54) for producing a cell culture mixture therein, a centrifugal separator (2), and a light phase receptacle (56), wherein
the method (100) comprises steps of:
- conducting a cell culture mixture from the fermentor (54) to an inlet (20) of the centrifugal separator (2), and
in a first mode:
- separating the cell culture mixture into a light phase and a heavy phase in the centrifugal separator (2), and
- conducting, a flow of separated light phase from a light phase outlet (22) of the centrifugal separator (2) to the fermentor (54) and a separated heavy phase from a heavy phase outlet (24) of the centrifugal separator (2) to the fermentor (54), and
in a second mode:
- separating the cell culture mixture into a light phase and a heavy phase in the centrifugal separator (2), and
- conducting, the flow of separated light phase from the light phase outlet (22) to the light phase receptacle (56) and the separated heavy phase from the heavy phase outlet (24) to the fermentor (54).

15. The method (100) according to claim 14, comprising a step of:
- conducting, in a third mode, a flow of cell culture mixture from the light phase outlet (22) to the fermentor (54) and a flow of cell culture mixture from the heavy phase outlet (24) to the fermentor (54).

16. The method (100) according to claim 15, wherein in the third mode, the centrifugal separator (2) is operated at a low rotational speed, such as below 50 rpm, and optionally a rotational direction of the rotor (4) of the centrifugal separator (2) is altered at least once.

17. The method (100) according to any one of claims 14 - 16, wherein the centrifugal separator (2) is a centrifugal separator (2) according to any one of claims 1 - 8 or a centrifugal separator (2) comprising an exchangeable separation insert (42) according to claim 9.
